# EUROPEAN PATENT APPLICATION

(11) **EP 0 673 655 A1**
(43) Date of publication of application: **27.09.1995**
(21) Application number: 93906789.8
(22) Date of filing: 18.03.1993
(51) Int. Cl.: A61K 49/00

(54) **MRI CONTRAST MEDIUM AND DIAGNOSTIC METHOD**

(30) Priority: 19.03.1992 JP 93561/92
(71) Applicant: DAIKIN INDUSTRIES, LIMITED, Osaka-shi Osaka 530 (JP)
(72) Inventor: YOSHIKAWA, Kohki, Tokyo 165 (JP); SHIONO, Takahiro, Kanto Rohsai Hospital, Kawasaki-shi, Kanagawa 211 (JP); IWAI, Hiroyuki, Yodogawa Works of Daikin Ind.Ltd.s, Settsu-shi, Osaka 566 566 (JP); YAMASHITA, Tsuneo, Yodogawa Works, Settsu-shi, Osaka 566 566 (JP); SHIMOKAWA, Kazuhiro, Yodogawa Works, Settsu-shi, Osaka 566 (JP)
(74) Representative: TER MEER - MÜLLER - STEINMEISTER & PARTNER
(86) International application number: JP9300322
(87) International publication number: WO9318795

(57) **Abstract**

An MRI contrast medium (suspension) comprising an oily fatty acid, fatty acid ester or perfluorinated compound and a particulate paramagnetic compound contained therein. It can be used stably for long and can be administered to a target region, whereby its dose can be reduced. In conducting diagnosis, it can be held in a target region stably for long, so that it makes monitoring by MRI possible and can dispense with X-ray CT, thus being free from the problem of radiation exposure.

## Description

### Industrial Field

This invention relates to an MRI contrast agent with the aims of selective administration to a targeted region; causing the agent to stably remain for a long time; enhancing diagnosis accuracy, which is conventionally executed by X-ray, by executing diagnosis by MRI; and reducing the burden on the patient by drastically reducing the number of X-ray exposure times, and also related to a method for diagnosis that utilizes the said MRI contrast agent.

### Prior Art

MRI (nuclear magnetic resonance imaging) observes nuclear magnetic resonance signals which are obtained from protons (water and fat in particular) in a living body, and it forms a contrast of the image by utilizing the proton density of each tissue and the difference between spin-lattice relaxation time (T1) and spin-spin relaxation time (T2).

Paramagnetic compounds become MRI contrast agent that enhance the contrast of an image by shortening the proton relaxation time. As one of these compounds, Gd-DTPA dimeglumine salt (registered trademark "Magnevist": Schering AG) is available in the market as the first MRI contrast agent in the world.

In general, the MRI contrast agent is intravenously administered as an aqueous solution. It is distributed to the outside of cells, the contrast effect disappears within tens of minutes, and the MRI contrast agent is then excreted through the kidneys. It is used for diagnosis of infarcts and tumors in the brain and the medulla spinal area due to its pharmacokinetics.
Furthermore, gut contrast agent of high concentration that can be administered perorally were recently developed and their clinical examination is in progress.

MRI contrast agent containing heavy metals, however, were of an aqueous solution formulation of a water soluble metal complex to enhance external excretability from the aspect of toxicity.

Accordingly, MRI contrasting was difficult to do in regions where contrast agent are easily excreted, such as the lymphatic area. Therefore, X-ray contrasting using an oily X-ray contrast agent (Lipiodol), which permits contrasting in a short time, was mainly used.

A chemical embolic therapy to execute treatment of a tumor by injecting in an artery an oily X-ray contrast agent (Lipiodol) alone or with an antitumor drugs suspended in it and by conducting embolization using a gelatin sponge was recently introduced (Takamasa Ryu, Innervision, 4 (1), 50 (1989)). This method achieved good treatment results due to the combination of blood inhibition by artery embolization and the antitumor drugs' staying effect. Also, the presence of the X-ray contrast agent is useful regarding diagnosis for observing progress and for discovering new tumors by X-ray diagnosis.

However, as the use of X-ray contrasting methods, X-ray computer tomography in particular, for image diagnosis expands, the issue of exposure cannot be ignored. Moreover, the X-ray contrasting method has such a disadvantage that long-time and highly frequent usage is restricted.

As a recent attempt to use an MRI contrast agent, injection of an emulsion of Lipiodol and registered trademark Magnevist, which is an aqueous solution, in an artery was performed (Michito Ishio, Japan Medical College Magazine, 58 (3), 285 (1911)). It was described, however, that separation occurred about 5 minutes later, and a stable formulation that permits use as an MRI contrast medium for long-time monitoring has not yet been reached.

### Objects of the invention

For these reasons, needs are rising for an MRI contrast agent that permits contrasting of regions such as arteries, lymphatics and uterotubes while maintaining low toxicity.

The MRI contrast agent of this invention is obtained by dispersing and suspending in an oily liquid fine particles of a paramagnetic compound of a quantity that produces sufficient contrast with MRI and provides stable formulation for a long time.

In addition, the said contrast agent can be selectively injected in a targeted region (such as an artery, lymphatics and uterotube) under fluororoentgenography in the case where the oily liquid has an X-ray shielding effect. Furthermore, since it is locally administered, it is possible to reduce the administration rate of the paramagnetic heavy metal compound.

Furthermore, because the agent stays in the tissue after being administered, it becomes possible to make many observations of the progress over a long period of time with only one administered dose, and since MRI is implemented for diagnosis at such observations, X-ray exposure, which was a problem with conventional X-ray computer tomography, can be avoided.

In addition, it is possible to monitor treatment by chemical embolic therapy by administering to a blocked region.

### Constituents of the invention

This invention related to an MRI contrast agent that is composed of an oily substance (fatty acid, fatty acid ester or perfluoro compound in particular) containing a fine-particle paramagnetic compound.

It is recommended that the said paramagnetic compound is a complex or a complex salt compound of at least one kind of metal selected from metals of atomic numbers 21-29, 42, 44 and lanthanum type metals of atomic numbers 58-70. In this case, the ligands may be ethylenediamine-N, N', N'', N'''-tetraacetic acid (hereinafter abbreviated as EDTA) diethylenetriamine-N, N', N'', N''', N''''-pentaacetic acid (hereinafter abbreviated as DTPA), 1, 4, 7, 10-tetraazacyclo dodecane-N, N', N'', N'''-tetraacetic acid (hereinafter abbreviated as DOTA), or 1, 4, 7, 10-tetraazacyclo dodecane-N, N', N''-triacetic acid (hereinafter abbreviated as DO3A).

The said fine-particle paramagnetic compound may be ammonium iron citrate (III), iron sulfate, or iron oxide.

The fine-particle paramagnetic compound of this invention is obtained by wet fine pulverization or dry fine pulverization to a particle size of 1-150µm or preferably 10-75µm.

With the said fatty acid, the lower alkyl ester of fatty acid, and the said perfluoro compound, it is preferable that the number of carbons of the fatty acid or the number of carbons of fatty acid of the lower alkyl ester of fatty acid is 4-30, the number of carbons of the lower alkyl is 1-4, and the number of carbons of the perfluoro compound is 1-12.

In the case where an oily liquid having an X-ray shielding effect is used, it is favorable to use a fatty acid or the lower alkyl ester of fatty acid with carbon numbers 4-30 substituted by one or more I or Br, or a normal chain alkyl perfluoro compound with carbon numbers 1-12 substituted by 1-10 I or Br.

Oily liquids having an X-ray shielding effect are easily available. Examples are iodinated poppy seed oil (registered trademark Lipiodol, etc.) and iodinated poppy seed oil fatty acid ethyl ester (registered trademark Lipiodol Ultra Fluid, etc.) which have been generally used as lymphatics contrast media and uterotube contrast media since they were developed in 1922 and the safety of which has been confirmed, and compounds of confirmed safety against living bodies out of normal chain saturated perfluoro compounds substituted by bromine or iodine such as perfluoro octyl bromide (PFOB).

The suspension of this invention is a metallic complex or a salt of the metallic complex composed of at least one type of metal selected out of known (described in, for example, Gazzette Specification disclosed as Patent Opening No.29718/1983) lanthanum type metals of atomic numbers 21-29, 42, 44, or 58-70 and ligands EDTA, DTPA, DOTA or DO3A, or includes one type of a paramagnetic compound obtained by preparing by a known method known (described in, for example, Gazzette Specification disclosed as Patent Opening No.191229/1990) ammonium iron citrate (III), iron sulfate or saccharated iron oxide.

The suspension of this invention is compounded by a paramagnetic compound of 10µmol-10 mmol, favorable 50-500 µmol per ml of oily liquid.

Various usually known additives such as a suspending agent, dispersing agent, thickening agent, etc. may be suitably added to this suspension as required.

For example, sodium carboxymethyl cellulose, carboxyvinyl polymer, glycerin fatty acid ester, polyvinylpyrrolidone, polyvinyl alcohol, macrogol, monooleic acid polyoxyethylene sorbitan, mono fatty acid glycerin, sodium lauryl sulfate, liquid paraffin, etc. may be indicated as suspending agent, dispersing agent or thickening agent. Olive oil, cetanol, tragacanth), etc. may be indicated as suspending agent or thickening agent. Furthermore, aluminum-magnesium silicate, saccharose fatty acid ester, sorbitan fatty acid ester, polyoxyethylene polyoxypropylene glycol, polyoxyethylene hardened castor oil, fatty acid polyoxyl, etc. may be indicated as suspending agent or dispersing agent, and suitable quantities can be used according to properties of formulation, production method and other factors considered.

The method for production of the suspension of this invention may be basically the same as the method for production of formulation such as the usual suspension. That is, production is possible in such a manner that the raw material, which is in a solid form and either dry pulverized using a grinder and classified or wet pulverized together with an oily compound as the base, is then weighed and blended with other components and is filled in ampoules, vials, etc.

The MRI contrast agent of this invention obtained in such a manner can also hold the nature of an X-ray contrast agent, and accordingly, administration to regions where dosage of an oily X-ray contrast agent has been applied such as arteries, lymphatics and uterotubes is possible, and the administration method is by injection, for instance. As the administration rate is suitably selected in correspondence to the region or tissue to be contrasted, the quantity of use can be reduced, and problems caused by heavy metals can be reduced. In the case of uterotube contrasting, for example, injection of 5-8 ml of the contrast agent of this invention is satisfactory.

Since the contrast agent of this invention is stable, it provides excellent preservability, and it can also be used in regions where excretion easily occurs. Furthermore, the contrast agent of this invention that is used by letting the agent stay is gradually separated, discharged, and then excreted, and accordingly, the degree of safety to living bodies is high.

In addition, the contrast agent of this invention can be selectively injected in the targeted regions or other internal organs under fluororoentgenography because the oily liquid has an X-ray shielding effect at the time of dosage administration. A diagnosis method for log-term monitoring using MRI equipment can be implemented after administration, In other words, use of an X-ray can be avoided. This is an extremely advantageous point.

### Industrial Application

The MRI contrast agent of this invention is produced by suspending a fine-particle paramagnetic compound in oily fatty acid, fatty acid ester, or perfluoro compound, and therefore, the agent can be used stably for a long time, and its administration rate can be reduced because administration can be made in the targeted region. Monitoring with MRI can be made on the occasion of diagnosis due to the agent's ability to stably remain for a long time in the targeted region. Thus, X-ray CT becomes unnecessary and the problem of exposure does not exist.

### Brief description of the drawings

Figure 1 is an MRI photo taken before administration of the contrast agent of this invention.

Figure 2 is an MRI photo taken immediately after administration of the said contrast agent.

Figure 3 is an X-ray CT photo taken immediately after administration of the said contrast agent and its sketch.

Figure 4 is an MRI photo taken two days after administration of the said contrast agent.

Figure 5 is an MRI photo taken seven days after administration of the said contrast agent.

Figure 6 is a graph that indicates the measurement result of the Gd-DTPA dissolution rate.

### Embodiments

This invention is explained in further detail below with embodiments.

### Embodiment 1

MRI contrast agent using dimeglumine salt of gadolinium of dietylenetriamine-N, N', N'', N''', N''''-pentaacetic acid (hereinafter abbreviated as Gd-DTPA):

Preparation of Gd-DTPA-registered trademark Lipiodol Ultra Fluid suspension (MRI contrast medium): Gd-DTPA dimeglumine salt 10g prepared by a known method was crushed in a mortar and was then classified to particle size of 200 mesh (75 µm) or less using a sieve made of stainless steel.

Iodinated poppy seed oil fatty acid ethyl ester (registered trademark Lipiodol Ultra Fluid) 10.0ml was added to this Gd-DTPA dimeglumine salt 1,195mg (1.25 mmol as one hydrate), and a suspension was produced by thoroughly mixing the two in a mortar. The suspension was an opaque light yellow oily substance.

### Embodiment 2

### Preparation of Gd-DTPA-PFOB suspension:

Except for use of PFOB as the oily compound, preparation of a suspension was made in correspondence to Embodiment 1. The obtained suspension was an opaque white liquid.

### Embodiment 3

### Contrasting testing:

The equipment used for this testing was X-ray CT-IMAGEMAX2 (slice thickness was 10mm) made by Yokogawa Medical Corp. and MRI-MAGNETON H15 (1.5T) made by Siemens Asahi Corp. The image pickup conditions of MRI were a T1 emphasized image of a spin echo method (SE 500/15) of a repeating time (TR) of 500 msec and an echo time (TE) of 15 msec, a slice thickness of 5mm, and the number of picture elements being 192 x 256.

VXII tumor cells were transplanted in the femoral region of a Japanese white rabbit (2.5-2.6kg) and were allowed to breed for ten days. General anesthesia was applied to the rabbit on the tenth day by a dosage of 30% urethane to the ear vein to suppress bodily motions, and MRI was implemented under anesthesia before an artery injection.

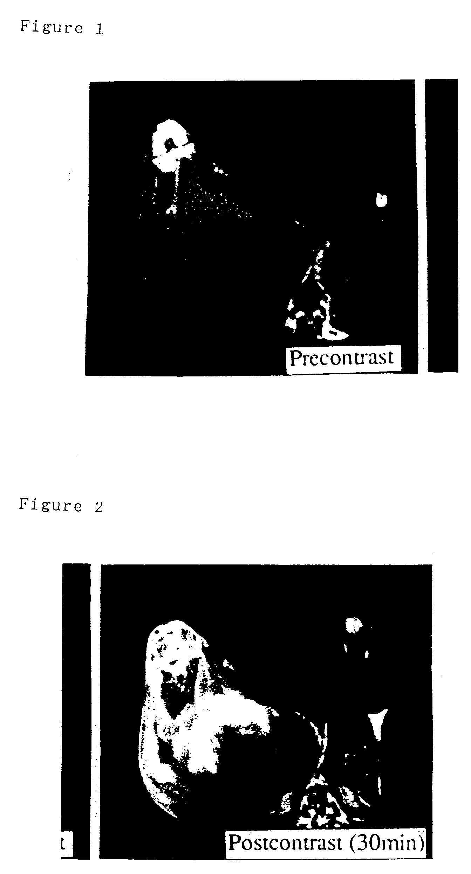

The femoral region was cut while the rabbit was under anesthesia and an indwelling needle was secured toward the end side of the femoral artery. Of the above-mentioned prepared suspension, 1.0ml was administered to the artery through the indwelling needle, and then flushing was performed using 5.0ml of saline solution and the artery was ligated. MRI and the X-ray CT (only immediately after administration) were applied immediately after suture of the femoral region, the second day, and the seventh day under the conditions stated above. Photos taken on these occasions and a sketch are shown in Figures 1-5.

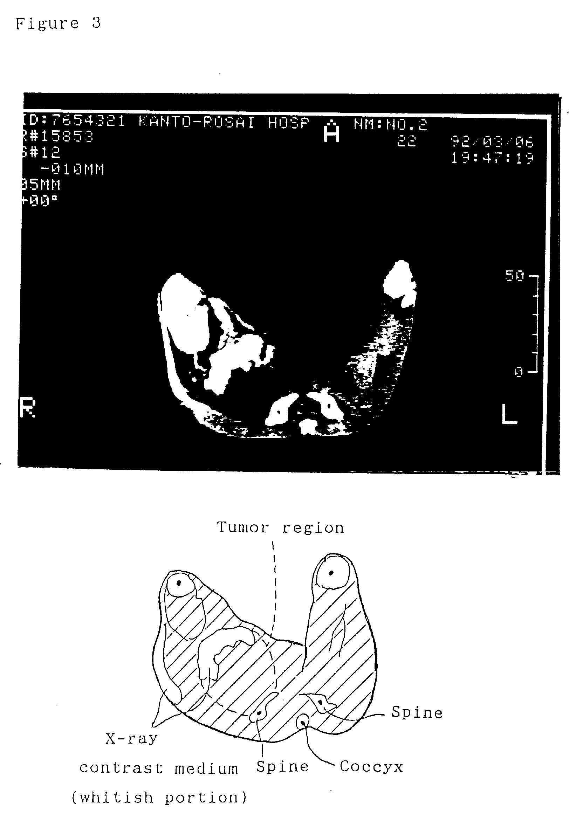

It is learned from these photos and the sketch that the contrasting action is stably maintained. Furthermore, it is satisfactory if the X-ray CT is applied immediately after administration, and MRI only may be applied thereafter. When Figures 1-5 are explained in detail, Figure 1, first of all, indicates an MRI photo of the above-mentioned femoral region before administration of the suspension as stated above. Compared to this photo, as is shown in the MRI photo in Figure 2 taken immediately after administration of the said suspension, the whitish contrast agent (MRI contrast agent ) is seen in the tumor region (see Figure 3). Since this MRI contrast agent is located in almost the same position as the X-ray contrast medium observed by X-ray CT shown in Figure 3 (sketch), it is learned that it is maintained in the targeted region without separation from the X-ray contrast agent.

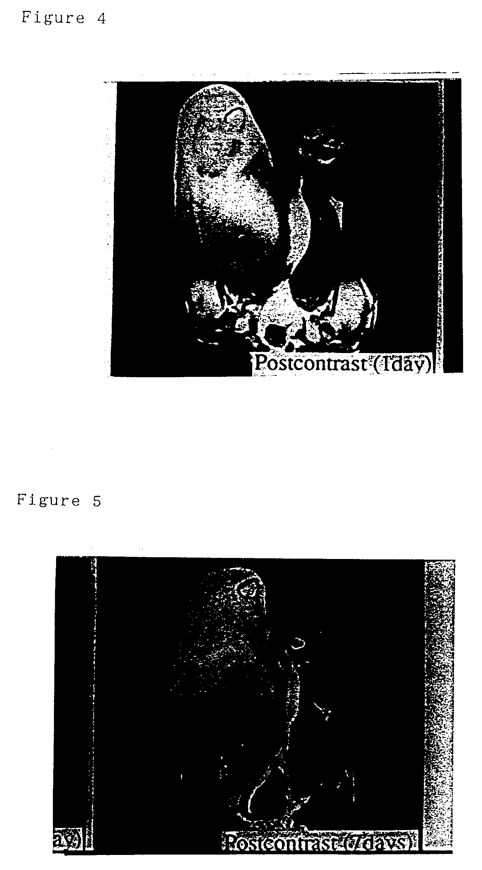

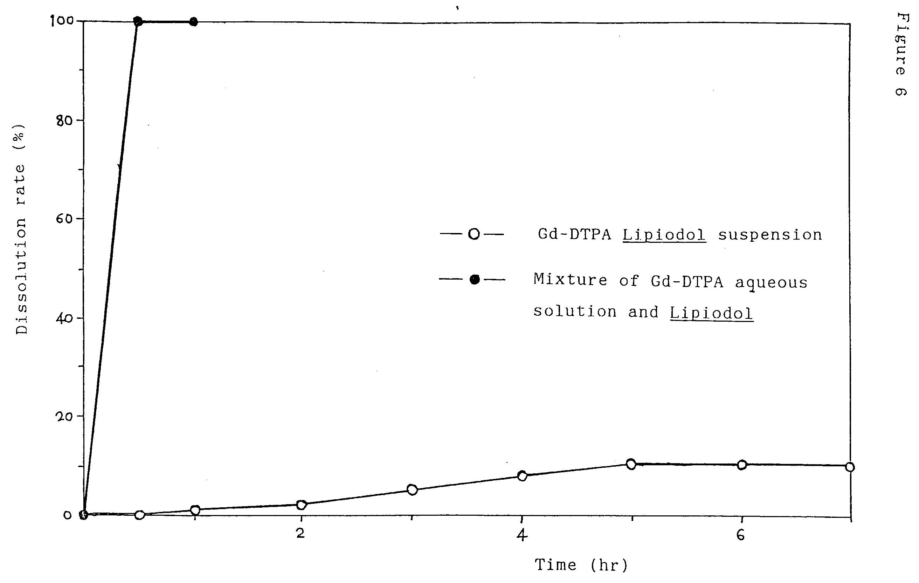

Next, on the second day after dosage of the suspension stated above, it can be confirmed that the whitish portion having the MRI contrast agent was expanded by immersion of the MRI contrast agent as shown in the MRI photo in Figure 4. (The cutting angle of this figure is slightly different from that of Figures 1 and 2). Furthermore, on the seventh day, the MRI contrast agent is still maintained and a necrosis portion in the tumor is seen as a blackish form in the MRI photo in Figure 5 (taken at a cutting angle identical to that of Figure 4). The suspension stated above can also be used for observation of such a necrosis portion.

### Embodiment 4

### Acute toxicity testing:

An amount of 12-600 µl of the suspension prepared as described above was administered to the abdominal cavity of a mouse MCH-ICR (35-40g), observation was made for seven days, a 50% lethal concentration (LD ) was checked, and the following data was obtained. It is learned from this data that the suspension of this invention is extremely safe.

| Suspension | LD |
|---|---|
| Gd-DTPA-registered trademark Lipiodol Ultra Fluid | 2.5 mmol/kg or more |

### Embodiment 5

### Stability of suspension:

To check the stability of the suspension prepared in Embodiment 1, the suspension was left at room temperature and the length of time until it completely separated into two layers was measured. Another suspension was prepared by uniformly mixing 1ml of 0.5 mmol/ml Gd-DTPA aqueous solution and 1ml registered trademark Lipiodol Ultra Fluid and was used for comparison. The result is shown below. It is learned form the result shown below that the suspension based on this invention is extremely stable.

| | | Length of time required for separation |
|---|---|---|
| suspension of Embodiment 1 | Gd-DTPA (solid) + Lipiodol Ultra Fluid | 2 days or longer |
| Suspension for comparison | Gd-DTPA (aqueous solution) + Lipiodol Ultra Fluid | 10 minutes |

### Embodiment 6

### Measurement of Gd-DTPA dissolution rate:

A dissolution test was conducted in correspondence with the dissolution test method specified in Revision 12 of the Japanese Pharmacopoeia to observe the behavior of dissolution of Gd-DTPA dimeglumine in water using the suspension prepared in Embodiment 1.

An amount of 20ml of the suspension was slowly added to 500ml of 37°C pure water and was stirred with a paddle at a speed of 100 rpm while the temperature was maintained at 37°C. Test liquids were sampled after 30 minutes and once every hour for one-seven hours, and the absorbance of 274nm was measured. The measured values of test liquids are shown by percentage based on the absorbance of 5mM Gd-DTPA aqueous solution as 1. The mixture for comparison described in Embodiment 5 was used for comparison. The result is shown in Figure 6.

It is learned form this result that the suspension based on this invention has characteristic of allowing extremely slow discharge of Gd-DTPA dimeglumine (slow discharge property).

## Claims

1. An MRI contrast agent composed of an oily substance that contains a fine-particle paramagnetic compound.

2. An MRI contrast agent described in Claim 1 wherein the fine-particle paramagnetic compound is a complex or complex salt compound of at least one type of metal selected from metals of atomic numbers 21-29, 42, and 44 and lanthanum type metals of atomic numbers 58-70.

3. An MRI contrast agent described in Claim 2 wherein ligands of the metallic complex or complex salt compound are ethylene diamine-N, N', N'', N'''-tetraacetic acid, diethylene- triamine-N, N', N'', N''', N''''-pentaacetic acid, 1, 4, 7, 10-tetraazacyclo dodecane-N, N', N'', N'''-tetraacetic acid, 1, 4, 7, 10-tetraazacyclo dodecane-N, N', N''-triacetic acid.

4. An MRI contrast agent described in any one of Claims 1-3 wherein the fine-particle paramagnetic compound is ammonium iron citrate (III), iron sulfate or iron oxide.

5. An MRI contrast agent described in any one of Claims 1- 4 wherein the oily substance is fatty acid, the lower alkyl ester of fatty acid, or a perfluoro compound.

6. An MRI contrast agent described in Claim 5 wherein the number of carbons of the fatty acid or the number of carbons of fatty acid of the lower alkyl ester of fatty acid is 4-30 and the number of carbons of the perfluoro compound is 1-12.

7. An MRI contrast agent described in Claim 5 wherein the fatty acid, the lower alkyl ester of fatty acid, or the perfluoro compound is substituted by iodine or bromine, the number of the carbons of the fatty acid or the number of carbons of fatty acid of the lower alkyl ester of fatty acid is 4-30, the number of carbons of the said lower alkyl is 1-4, and the number of carbons of the perfluoro compound is 1-12.

8. A diagnosis method utilizing the MRI contrast agent described in any of Claims 1-7 and monitoring the administered region using MRI equipment.
